# EUROPEAN PATENT APPLICATION

(11) **EP 4 306 633 A1**
(43) Date of publication of application: **17.01.2024**
(21) Application number: 22767125.2
(22) Date of filing: 08.03.2022
(51) Int. Cl.: C12N 5/073, C12N 5/071

(54) **CELL CLUSTER PRODUCTION METHOD**

(30) Priority: 09.03.2021 JP 2021037339
(71) Applicant: National University Corporation Tokyo Medical and Dental University, Bunkyo-ku Tokyo 113-8510 (JP); Takeda Pharmaceutical Company Limited, Osaka-shi, Osaka 541-0045 (JP)
(72) Inventor: TAKEBE Takanori, Tokyo 113-8510 (JP); SAIKI Norikazu, Tokyo 113-8510 (JP)
(74) Representative: Jones, Nicholas Andrew
(86) International application number: PCT/JP2022/009925
(87) International publication number: WO 2022/191171

(57) **Abstract**

The present invention provides the following method as an approach for enriching desired cells, particularly, yolk sac mesoderm cells or amniotic ectoderm cells, contained in an embryogenesis region-specific micropattern structure of a cell cluster formed by differentiation-inducing factors from a colony of pluripotent stem cells such as iPS cells: a method for producing a cell cluster enriched in desired cells from pluripotent stem cells, comprising the step of two-dimensionally culturing the pluripotent stem cells under Wnt signal regulation. The present invention provides, for example, a method for producing a cell cluster enriched in yolk sac mesoderm cells from pluripotent stem cells, comprising the step of two-dimensionally culturing the pluripotent stem cells under conditions that activate Wnt signals, and a method for producing a cell cluster enriched in amniotic ectoderm cells from pluripotent stem cells, comprising the step of two-dimensionally culturing the pluripotent stem cells under conditions where Wnt signals have been suppressed.

## Description

### Technical Field

The present invention relates to a method for producing a cell cluster enriched in desired cells, the cell cluster having an embryogenesis region-specific micropattern formed by differentiation-inducing factors from a colony of pluripotent stem cells. The present invention also relates to a method for producing a cell population that is formed by further allowing cells in the cell cluster to differentiate, and an organoid or a three-dimensional organ that is formed by further culturing cells in the cell cluster or cells in the cell population.

### Background Art

Pluripotent stem cells such as ES cells or iPS cells correspond to epiblast according to embryologic definition. It is known that a cell cluster having an embryogenesis region-specific micropattern is formed by differentiation-inducing factors from a colony of pluripotent stem cells. For example, Non Patent Literature 1 states that the two-dimensional culture of human ES cells formed a micropattern containing three germ layers (endoderm, mesoderm, and ectoderm) by BMP4 signals. Also, Non Patent Literature 2 states that after formation of a micropattern from human ES cells, differentiation into endoderm and mesoderm was controlled by inhibiting Wnt signals. Non Patent Literature 3 states that in the case of forming a micropattern from mouse ES cells, yolk sac mesoderm cells also appeared, albeit at a low proportion.

Meanwhile, mesoderm cells further differentiate into vascular endothelial cells and the like. For example, Patent Literature 1 describes a matrix composition production method of forming a hierarchical cell network (e.g., a vascular or neural network) by gas-liquid interface culture, and states that vascular endothelial cells are used as vascular cells for forming the cell network. Examples of Patent Literature 1 state that lateral plate mesodermal cells were induced from human iPS cells by culture using a medium containing BMP4, VEGF, CHIR99021, and the like, and hemogenic endothelial cells (CD34-positive and CD73-negative) were obtained by further culture after medium replacement; and a matrix composition was produced by forming a hierarchical cell network using plural types of cells including the hemogenic endothelial cells thus obtained.

### Citation List

### Patent Literature

Patent Literature 1: WO2020/203713

### Non Patent Literature

Non Patent Literature 1: Warmflash et al., NATURE METHOD VOL.11 No.8 August 2014
Non Patent Literature 2: Martyn et al., Development (2019) 146, dev17291
Non Patent Literature 3: Morgani et al., eLife 2018; 7: e32839

### Summary of Invention

### Technical Problem

A method for adjusting the ratio of particular cells contained in an embryogenesis region-specific micropattern structure, for example, a method for enriching particular cells and allowing the cells to differentiate to efficiently obtain cells of interest, has not yet been known about a cell cluster formed by differentiation-inducing factors from a colony of pluripotent stem cells.

An object of the present invention is to provide an approach for enriching desired cells, particularly, yolk sac mesoderm cells (in the present specification, also referred to as "YSMCs") or amniotic ectoderm cells (in the present specification, also referred to as "AECs"), contained in a cell cluster formed from pluripotent stem cells.

### Solution to Problem

The present inventors have found that desired cells in a cell cluster can be enriched by two-dimensionally culturing pluripotent stem cells under Wnt signal regulation, for example, a cell cluster enriched in YSMCs can be produced by two-dimensionally culturing pluripotent stem cells under conditions that activate Wnt signals, such as the addition of a Wnt signal activator to a medium at the start of culture of the pluripotent stem cells, whereas a cell cluster enriched in AECs can be produced by two-dimensionally culturing pluripotent stem cells under conditions where Wnt signals have been suppressed, such as the addition of a Wnt signal inhibitor to a medium at the start of culture of the pluripotent stem cells. In one aspect of the cell cluster, an important feature is that desired cells such as YSMCs or AECs and other cells form a layered structure while keeping their two-dimensional positional relationship and boundary; and the thickness of a layer containing the desired cells can be increased.

Specifically, the present invention includes at least the following items.
[1] A method for producing a cell cluster enriched in desired cells from pluripotent stem cells, the method comprising the step of two-dimensionally culturing the pluripotent stem cells under Wnt signal regulation.
[2] The method according to [1], wherein the desired cells are yolk sac mesoderm cells, and the step of two-dimensionally culturing the pluripotent stem cells under Wnt signal regulation is a Wnt signal-activating culture step of two-dimensionally culturing the pluripotent stem cells under conditions that activate Wnt signals.
[3] The method according to [2], wherein the Wnt signal-activating culture step is the step of two-dimensionally culturing the pluripotent stem cells using a medium supplemented with a Wnt signal activator.
[4] The method according to [3], wherein the Wnt signal activator is a GSK3 inhibitor.
[4a] The method according to [4], wherein the GSK3 inhibitor is CHIR99021.
[5] The method according to [2], wherein the Wnt signal-activating culture step is performed at the start of culture of the pluripotent stem cells.
[6] The method according to [1], wherein the pluripotent stem cells are induced pluripotent stem cells.
[7] A cell population production method further comprising a culture step of allowing the obtained yolk sac mesoderm cells to differentiate into CD34+ vascular endothelial progenitor cells after the Wnt signal-activating culture step according to [2].
[8] The method according to [7], further comprising a culture step of allowing the CD34+ vascular endothelial progenitor cells to differentiate into CD34+CD32+ vitelline venous hemogenic endothelial cells.
[9] A cell cluster obtained by the method according to [1].
[10] A cell population obtained by the method according to [7].
[11] An organoid or three-dimensional organ production method comprising the step of three-dimensionally culturing at least a portion of the cell cluster according to [9] or the cell population according to [10].
[12] An organoid or a three-dimensional organ obtained by the production method according to [11].
[13] The method according to [1], wherein the desired cells are amniotic ectoderm cells, and the step of two-dimensionally culturing the pluripotent stem cells under Wnt signal regulation is a Wnt signal-suppressing culture step of two-dimensionally culturing the pluripotent stem cells under conditions where Wnt signals have been suppressed.
[14] The method according to [13], wherein the Wnt signal-suppressing culture step is performed using a medium supplemented with a Wnt signal inhibitor.
[15] The method according to [14], wherein the Wnt signal inhibitor is IWP-2.

### Advantageous Effects of Invention

According to the present invention, a cell cluster enriched in desired cells can be produced from pluripotent stem cells by two-dimensionally culturing the pluripotent stem cells under Wnt signal regulation. Particularly, a cell cluster enriched in YSMCs can be produced by a Wnt signal-activating culture step, and a cell cluster enriched in AECs can be produced by a Wnt signal-suppressing culture step. For example, CD34+ vascular endothelial progenitor cells which can be used in the production of an organoid or a three-dimensional organ can be efficiently produced from the cell cluster enriched in YSMCs.

### Brief Description of Drawings

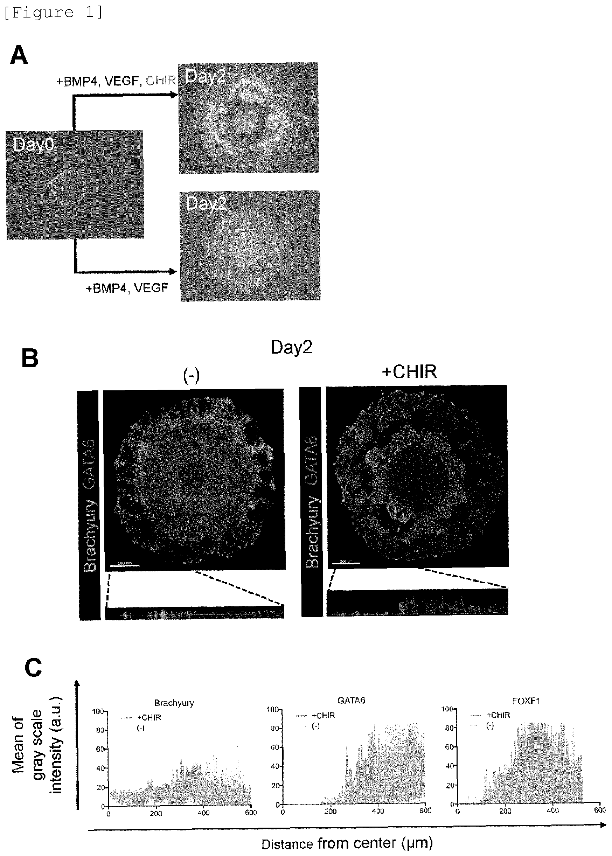

[Figure 1] Figure 1 relates to [1] "Preparation of human yolk sac mesoderm cells by Wnt signal-activating culture step (first embodiment of the present invention)" and [3] "Differentiation marker analysis of cell population by immunocytostaining" in Examples. "+CHIR" depicts the case where CHIR99021 (Wnt signal activator) was added to a medium, and "(-)" depicts the case where no CHIR99021 was added as a control. [Figure 1A] Images observed under an optical microscope. The right upper box is an image on 2 days after the start of culture of human iPS cells under Wnt signal-activating conditions (where the Wnt signal activator was added to a medium), and the right lower box is an image on 2 days after the start of culture of human iPS cells under Wnt signal non-activating conditions (where no Wnt signal activator was added to a medium) as a control. [Figure 1B] Fluorescent images of Brachyury and GATA6. In color images, the central part of a cell cluster is stained green which indicates the expression of Brachyury, and the outer edge part thereof is stained purple which indicates the expression of GATA6. [Figure 1C] Graphs showing the relationship between the fluorescence intensity (in terms of a gray scale) of each of Brachyury, GATA6, and FOXF1 and a distance from the center in the respective fluorescent images of +CHIR and (-).

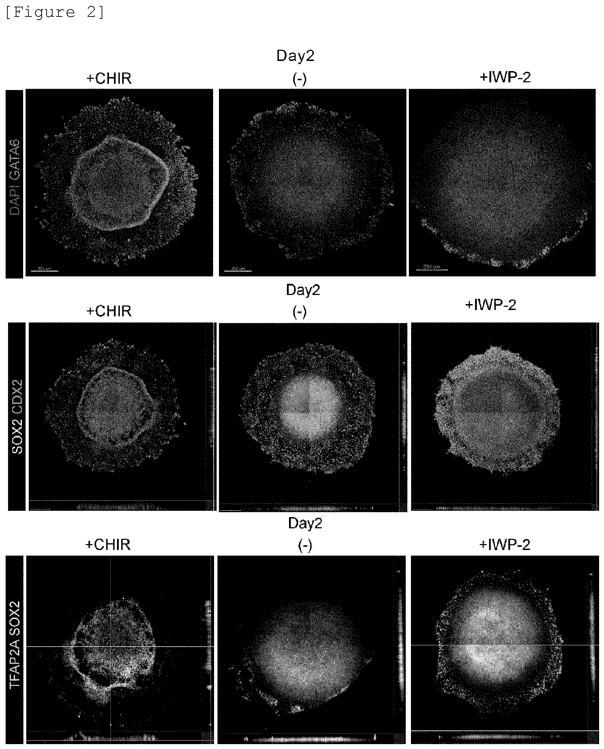

[Figure 2] Figure 2 relates to [2] "Preparation of human amniotic ectoderm cells by Wnt signal-inhibiting culture step (second embodiment of the present invention)" and [3] "Differentiation marker analysis of cell population by immunocytostaining" in Examples. "+IWP-2" depicts the case where IWP-2 (Wnt signal activator) was added to a medium; "(-)" depicts the case where no IWP-2 was added as a control; and "+CHIR" depicts the case where CHIR99021 used in the first embodiment of the present invention was added instead of IWP-2 to a medium for reference. Upper boxes: Fluorescent images of DAPI and GATA6. In color images, the central part of a cell cluster is stained blue with DAPI, and the outer edge part thereof is stained red which indicates the expression of GATA6. Middle boxes: Fluorescent images of SOX2 and CDX2. In color images, the central part of a cell cluster is stained cyan which indicates the expression of SOX2, and the outer edge part thereof is stained pink which indicates the expression of CDX2. Lower boxes: Fluorescent images of TFAP2A and SOX2. In color images, the central part of a cell cluster is stained cyan which indicates the expression of SOX2, and the outer edge part thereof is stained orange which indicates the expression of TFAP2A (mainly, +IWP-2).

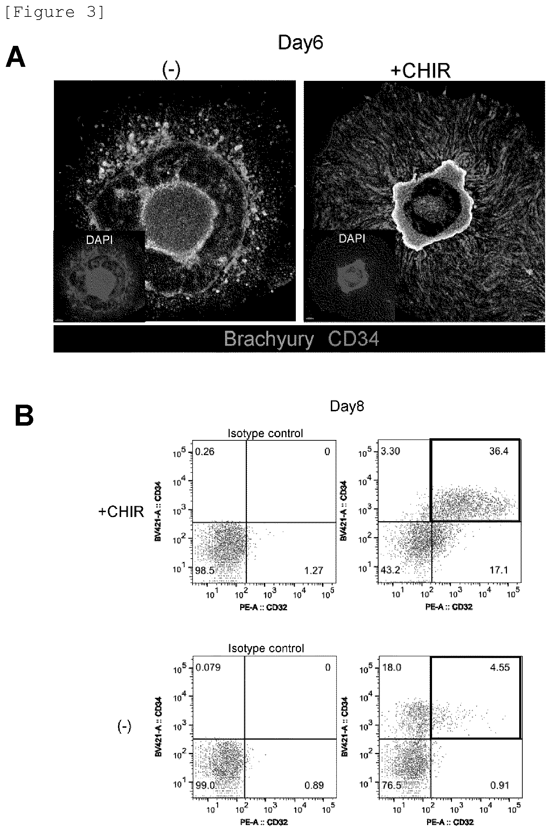

[Figure 3] Figure 3 relates to [4] "Preparation of human vitelline venous hemogenic endothelial cells", [5] Differentiation marker analysis of cell population by immunocytostaining, and [6] "Differentiation marker analysis using flow cytometry" in Examples. "+CHIR" depicts the case where a cell cluster obtained by adding CHIR99021 (Wnt signal activator) to a medium was induced to differentiate, and "(-)" depicts the case where a cell cluster obtained without the addition of CHIR99021 was induced to differentiate as a control.
[Figure 3A] Fluorescent images of Brachyury and CD34. In color images, a cell population of the right box (+CHIR) has a relatively small region of a green central part which indicates the expression of Brachyury, and a relatively largely expanded region of a red outer edge part which indicates the expression of CD34, whereas a cell population of the left box (-) has a relatively large region of a green central part which indicates the expression of Brachyury, and an outer edge part stained orange which indicates the expression of GATA6 and lower expression of Brachyury than that in the central part. [Figure 3B] Analysis results of flow cytometry based on the expression of CD34 and CD32.

### Description of Embodiments

### - Cell cluster production method -

The cell cluster production method of the present invention is a method for producing a cell cluster enriched in desired cells from pluripotent stem cells, comprising the step of two-dimensionally culturing the pluripotent stem cells under Wnt signal regulation. The cell cluster production method of the present invention representatively encompasses the following two embodiments, the first embodiment and the second embodiment.

In the present specification, the term "enriched" in desired cells means that the amount of the desired cells or the ratio of the amount of the desired cells to the amount of all cells in a cell cluster is increased in comparison with the amount or the ratio in a control such as a cell cluster before enrichment or a cell cluster obtained without carrying out the present invention (under conditions that do not regulate Wnt signals). In other words, the term "enrich" and "enrichment" for desired cells refers to increasing the amount of the desired cells or the ratio of the amount of the desired cells to the amount of all cells in a cell cluster is increased in comparison with the amount or the ratio in a control. In a particular embodiment of the present invention, the desired cells in the cell cluster produced according to the present invention are enriched such that the amount or the ratio thereof is increased by at least 20%, 30%, 40%, 50%, 60%, 70%, 80%, 90%, 100%, 120%, 150%, 200%, 300%, 400%, 500%, 600%, 700%, 800%, 900%, or 1000% in comparison with the amount or the ratio in a control cell cluster.

In the present specification, the term "two-dimensional culture" of pluripotent stem cells refers to feeder-free adhesion culture of the pluripotent stem cells. A culture method for two-dimensionally culturing (feeder-free adhesionculturing) pluripotent stem cells such as iPS cells or ES cells is known in the art. For example, a method for culturing human ES/iPS cells in a feeder-free manner is described in Rodin S et al., Nat Biotechnol. (2010) 28 (6): 611-5, Chen et al., Nat Methods (2011) 8 (5): 424-429, Miyazaki, T. et al. Nat Commun (2012) 3, 1236, Okita et al., Stem Cells, (2013) 31 (3): 458-66, and Nakagawa M et al., Scientific Reports, (2014) 4: 3594.

In the present specification, the "cell cluster" refers to a collection of cells having an embryogenesis region-specific micropattern formed from a colony of pluripotent stem cells. The "colony" refers to a visible clump originating from one cell in a solid medium. The "colony of pluripotent stem cells" refers to a visible cell collection resulting from the adhesion of cells replicated from one pluripotent stem cell that has self-replicated while maintaining its undifferentiated state. The "micropattern" refers to a state where different types of cells are arranged according to a given rule (the rule may occur naturally or may be artificially designed), not at random. The "embryogenesis region-specific micropattern" refers to a geometric arrangement partially similar to a cell differentiation pattern in embryo formation in a living body, wherein the geometric arrangement is formed from a cell collection that differentiates into plural types of cells while maintaining radiation symmetry. An approach for forming such a cell cluster from the colony of pluripotent stem cells, for example, differentiation-inducing factors to be added to a medium, is known in the art (see, for example, Non Patent Literatures 1 to 3 described above). In the present invention as well, the same or similar differentiation-inducing factors as conventional ones can be used in both the first embodiment and the second embodiment described below. Examples of such differentiation-inducing factors include BMP4, TGFb, bFGF, and VEGF. Particularly, BMP4 and VEGF are preferably used.

The amounts of the differentiation-inducing factors used are not particularly limited and can be appropriately adjusted by those skilled in the art according to the types of the differentiation-inducing factors in a Wnt signal-activating culture step or a Wnt signal-suppressing culture step, the combination of the differentiation-inducing factors or of the differentiation-inducing factors with a medium, etc. such that a cell cluster having a predetermined micropattern is formed and such that in the present invention, particularly, a cell cluster enriched in desired cells can be produced when a Wnt signal activator or a Wnt signal suppressor is further added. The concentration of BMP4 in a medium can be set to within the range of, for example, 10 to 100 µM, preferably 50 to 80 µM. The concentration of TGFb in a medium can be set to within the range of, for example, 2 to 20 µM, preferably 2 to 10 µM. The concentration of bFGF in a medium can be set to within the range of, for example, 10 to 100 µM, preferably 50 to 80 µM. The concentration of VEGF in a medium can be set to within the range of, for example, 5 to 100 µM, preferably 50 to 80 µM.

The "medium" for use in two-dimensionally culturing pluripotent stem cells under Wnt signal regulation can be basically the same as a general medium for use in the two-dimensional culture of pluripotent stem cells, particularly, a medium known in the art that is used for forming a cell cluster having an embryogenesis region-specific micropattern by the two-dimensional culture of pluripotent stem cells, except that a Wnt signal activator may be further added in the first embodiment; and a Wnt signal suppressor may be further added in the second embodiment. Those skilled in the art can prepare a suitable medium by selecting suitable types and amounts of a basal medium and an additive optionally used according to the pluripotent stem cells to be cultured, and mixing them.

Examples of the medium for pluripotent stem cells such as iPS cells or ES cells include DMEM, DMEM/F12, and DME culture solutions containing 10 to 15% FBS (these culture solutions can further appropriately contain LIF, penicillin/streptomycin, puromycin, L-glutamine, nonessential amino acids, β-mercaptoethanol, and the like) and commercially available culture solutions, for example, a culture solution for mouse ES cell culture (TX-WES culture solution, Thromb-X, LLC), a culture solution for primate ES cell culture (culture solution for primate ES/iPS cells, ReproCELL Inc.), a serum-free medium (mTESR, STEMCELL Technologies Inc.), a medium for iPS/ES cell proliferation/medium for regenerative medicine (StemFit(R) AK02N, Ajinomoto Healthy Supply Co., Inc.), and a feeder-free medium for iPS cell culture (Essential 8, Gibco). A serum-free medium may be used (e.g., Sun N, et al. (2009), Proc Natl Acad Sci USA. 106: 15720-15725).

The culture container is not particularly limited, and a dish, a flask, a microplate, a cell culture sheet such as trade name "OptiCell" (Nunc/Thermo Fisher Scientific Inc.), or the like can be used. The culture container is preferably surfacetreated in order to improve adhesiveness to cells (hydrophilicity), or coated with a substrate for cell adhesion such as collagen, gelatin, poly-L-lysine, poly-D-lysine, laminin, fibronectin, Matrigel (e.g., BD Matrigel (Nippon Becton Dickinson Co., Ltd.)), or vitronectin.

### First embodiment: Wnt signal-activating culture step

The first embodiment is a method for producing a cell cluster enriched in "yolk sac mesoderm cells" (YSMCs) as the "desired cells". In this embodiment, the "step of two-dimensionally culturing the pluripotent stem cells under conditions that activate Wnt signals" (Wnt signal-activating culture step) is performed as the "step of two-dimensionally culturing the pluripotent stem cells under Wnt signal regulation".

YSMCs are cells that differentiate into blood cells forming a blood island and cells constituting a vascular structure in yolk sac which is an extraembryonic hematopoietic organ during early development, and can be identified as cells positive to GATA6 and negative to Brachyury (GATA6+Brachyury-) as cell markers. In addition, YSMCs may be positive to FOXF1, GATA3, GATA4, fibronectin (FN1), collagen (e.g., COL1A1, COL1A2, COL3A1, COL4A1, COL6A1, and COL6A3), laminin-111 (e.g., LAMA1, LAMB1, and LAMC1), KDR, and HAND1, etc. Whether or not a cell cluster is enriched in YSMCs and the degree of enrichment can be quantitatively or qualitatively evaluated or determined, for example, by analyzing a fluorescent image obtained by immunostaining targeting such cell markers, and comparing the results with a control.

The Wnt signal-activating culture step is suitably performed at the start of culture (early stage of culture) for inducing the differentiation of pluripotent stem cells into a cell cluster having a predetermined micropattern, more specifically, before the differentiation of pluripotent stem cells into embryonic epiblast containing embryonic ectoderm and primitive streak, and extraembryonic ectoderm. For example, it is preferred to culture iPS cells under conditions that activate Wnt signals, i.e., the Wnt signal-activating culture step, on 0 to 2 days (Day 0 to Day 2) counted from the start of induction of differentiation by the culture of (a colony of) the iPS cells in a medium supplemented with predetermined differentiation-inducing factors. More specifically, it is preferred to add a Wnt signal activator (together with predetermined differentiation-inducing factors) to medium on Day 0 and culture pluripotent stem cells such as iPS cells in the medium. In such an embodiment, the addition of the Wnt signal activator to the medium on Day 1 and/or the medium on Day 2 is not essential.

The "conditions that activate Wnt signals" are not particularly limited as long as the working effect of the present invention is exerted by which a cell cluster enriched in YSMCs can be obtained. An approach known in the art can be used. Typically, examples of the conditions that activate Wnt signals include the addition of a type and an amount of a substance having an effect of activating Wnt signals, i.e., a "Wnt signal activator", to a medium.

The type of the Wnt signal activator is not particularly limited, and a GSK3 inhibitor, for example, is preferred. Examples of the GSK3 inhibitor include compounds such as CHIR99021, SB-216763, BIO (6-bromoindirubin-3'-oxime), and LY2090314. Further examples of the Wnt signal activator other than the GSK3 inhibitor include SFRP inhibitors (e.g., WAY-316606), Notum inhibitors (e.g., ABC99), PP2A activators (e.g., IQ1), ARFGAP1 activators (e.g., QS11), β catenin activators (e.g., DCA), and compounds such as (hetero)arylpyrimidine and 2-amino-4-[3,4-(methylenedioxy)benzyl-amino]-6-(3-methoxyphenyl)pyrimidine. In the method of the present invention, two or more, for example, two, three, or four types of Wnt signal activators, may be used in combination.

The amount of the Wnt signal activator used is not particularly limited and can be appropriately adjusted such that a desired degree of the working effect is exerted according to the type of the Wnt signal activator, i.e., a cell cluster is enriched in YSMCs to a desired degree. In the case of using, for example, a GSK3 inhibitor (CHIR99021, etc.) as the Wnt signal activator, the concentration of the GSK3 inhibitor in a medium can be set to within the range of, for example, 0.5 to 3 µM, preferably 1 to 2 µM.

A growth factor BMP4, which is routinely used for culturing iPS cells or the like (BMP4 is essential for inducing almost all differentiated cells from epiblast), is known to be related to Wnt signals. However, BMP4 used in a general amount for use in the culture of iPS cells or the like, i.e., in an amount where the working effect, i.e., the enrichment of YSMCs, of the first embodiment of the present invention is not observed (e.g., used as a control in Examples described later) is not regarded as the Wnt signal activator according to the first embodiment. In other words, the Wnt signal activator according to the present invention refers to a type and an amount of a substance, other than BMP4, which is found (e.g., by comparison with a control) to have the working effect, i.e., the enrichment of YSMCs, of the first embodiment of the present invention in itself, regardless of BMP4, even if BMP4 is present in a general amount in a medium.

### Second embodiment: Wnt signal-suppressing culture step

The second embodiment is a method for producing a cell cluster enriched in "amniotic ectoderm cells" (AECs) as the "desired cells". In this embodiment, the "step of two-dimensionally culturing the pluripotent stem cells under conditions where Wnt signals have been suppressed" (Wnt signal-suppressing culture step) is performed as the "step of two-dimensionally culturing the pluripotent stem cells under Wnt signal regulation".

AECs are ectodermal cells that differentiate into amniotic epithelial cells which surround embryo. CDX2, TFAP2A, GATA3, TFAP2B, E-cadherin, and the like are known as cell markers for AECs, and AECs can be identified from whether to be positive to one or two or more thereof (positive cells are AECs, while negative cells are not AECs). AECs can also be identified from whether to be negative to one or two or more of cell markers for undifferentiated cells or embryonic ectoderm, such as NANOG and SOX2 (negative cells are AECs, while positive cells are undifferentiated cells or ectoderm) in addition to the cell markers (positive markers) described above. Whether or not a cell cluster is enriched in AECs can be quantitatively or qualitatively evaluated or determined, for example, by analyzing a fluorescent image obtained by immunostaining targeting such cell markers, and comparing the results with a control.

The "conditions where Wnt signals have been suppressed" are not particularly limited as long as the working effect of the present invention is exerted by which a cell cluster enriched in AECs can be obtained. An approach known in the art can be used. Typically, examples of the conditions that suppress Wnt signals include the addition of a type and an amount of a substance having an effect of suppressing Wnt signals, i.e., a "Wnt signal suppressor", to a medium.

The type of the Wnt signal suppressor is not particularly limited, and a Porcupine (PORCN) inhibitor, for example, is preferred. Examples of the Porcupine inhibitor include compounds such as IWP-2, LGK974, Wnt-C59, ETC-159, IWP-O1, IWP L6, GNF-6231, and Porcn-IN-1. Further examples of the Wnt signal suppressor other than the Porcupine inhibitor include (non-membrane-bound) free Wnt inhibitors (e.g., Ant1.4Br/Ant 1.4Cl), Frizzled inhibitors (e.g., Niclosamide), Vacuolar ATPase inhibitors (e.g., apicularen and bafilomycin), tankyrase 1/Axin activators (e.g., XAV939), Axin activators (e.g., IWR), tankyrase, Axin activators (e.g., G007-LK and G244-LM), CK1 inhibitors (e.g., pyrvinium), Dsh inhibitors (e.g., NSC668036), TCF/β catenin inhibitors (e.g., 2,4-diamino-quinazoline and PKF115-584), TCF inhibitors (e.g., quercetin), CREB-binding protein inhibitors (e.g., ICG-001), β catenin-TBL interaction inhibitors (e.g., BC2059), and compounds such as Shizokaol D. In the method of the present invention, two or more, for example, two, three, or four types of Wnt signal suppressors, may be used in combination.

The amount of the Wnt signal suppressor used is not particularly limited and can be appropriately adjusted such that a desired degree of the working effect is exerted according to the type of the Wnt signal suppressor, i.e., a cell cluster is enriched in AECs to a desired degree. In the case of using, for example, a Porcupine inhibitor (IWP-2, etc.) as the Wnt signal suppressor, the concentration of the Porcupine inhibitor in a medium can be set to within the range of, for example, 0.5 to 2 µM, preferably 0.5 to 1 µM.

In the second embodiment of the present invention as well, BMP4 can be added in a general amount for use in the culture of iPS cells or the like to a medium. The Wnt signal suppressor according to the present invention refers to a type and an amount of a substance, other than BMP4, which is found (e.g., by comparison with a control) to have the working effect, i.e., the enrichment of AECs, of the second embodiment of the present invention even in such a case.

### Cells other than desired cells

The cell cluster obtained by the cell cluster production method of the present invention can contain cells other than the desired cells such as YSMCs or AECs (in the present specification, referred to as "non-desired cells"). Examples of the non-desired cells include cells, other than YSMCs and AECs, contained in micropatterns known in the art, for example, SOX2-positive embryonic ectoderm, T (Brachyury)-positive cells (primitive streak), and embryonic mesoderm and embryonic endoderm derived therefrom. In one aspect, a feature of the present invention is also that in the cell cluster obtained by the production method of the present invention, the desired cells such as YSMCs or AECs and the non-desired cells form a layered structure while keeping their two-dimensional positional relationship and boundary; and the thickness of a layer containing the desired cells can be increased.

### - Cell population production method -

The cell population production method of the present invention is a method that further comprises a culture step of allowing the obtained yolk sac mesoderm cells (YSMCs) to differentiate into CD34+ vascular endothelial progenitor cells (EPCs) (EPC differentiation step) after the Wnt signal-activating culture step in accordance with the first embodiment of the cell cluster production method, and can further comprise other steps, if necessary.

In the present specification, the "cell population" described, particularly, in relation to the production method refers to a collection of cells allowed to include cells differentiated from the desired cells (YSMCs, AECs, etc.) by further culture after formation of the "cell cluster" mentioned above.

EPCs can be identified as CD34-positive cells and are cells having the ability to differentiate into vascular endothelial cells (ECs). EC into which EPCs differentiate encompasses, for example, vitelline venous hemogenic endothelial cells (VVHECs, CD34-positive and CD32-positive) mentioned later as well as microvessel endothelial cells (MVECs) and umbilical-vein endothelial cells (UVECs). Also, EC into which EPCs differentiate encompasses both of hemogenic endothelial cells (HECs, CD34-positive and CD73-negative) which refer to vascular endothelial cells having hematopoietic capacity, and nonhemogenic endothelial cells (non-HEC, CD31-positive, CD73-positive, and CD144-positive) which refer to vascular endothelial cells having no hematopoietic capacity.

The medium for a pluripotent stem cell in the cell cluster production method of the present invention mentioned above, or a medium for a cell of interest into which pluripotent stem cells or the like differentiate, or a mixture thereof can be used as a medium in the cell population production method of the present invention. Examples of the basal medium for an EPC include DMEM/F-12 (Gibco), Stempro-34 SFM (Gibco), Essential 6 medium (Gibco), Essential 8 medium (Gibco), EGM (Lonza Group AG), BulletKit (Lonza Group AG), EGM-2 (Lonza Group AG), Bullet Kit (Lonza Group AG), EGM-2 MV (Lonza Group AG), VascuLife EnGS Comp Kit (LCT), Human Endothelial-SFM Basal Growth Medium (Invitrogen Corp.), and human microvessel endothelial cell proliferation medium (Toyobo Co., Ltd.). Examples of an additive for an EPC include one or more members selected from the group consisting of B27 Supplements (Gibco), BMP4 (bone morphogenetic protein 4), GSKβ inhibitors (e.g., CHIR99021), VEGF (vascular endothelial growth factor), FGF2 (fibroblast growth factor (also referred to as bFGF (basic fibroblast growth factor))), Folskolin, SCF (stem cell factor), TGFβ receptor inhibitors (e.g., SB431542), Flt-3L (Fms-related tyrosine kinase 3 ligand), IL-3 (interleukin 3), IL-6 (interleukin 6), TPO (thrombopoietin), hEGF (recombinant human epithelial growth factor), hydrocortisone, ascorbic acid, IGF1, FBS (fetal bovine serum), antibiotics (e.g., gentamycin and amphotericin B), heparin, L-glutamine, Phenol Red, and BBE.

The EPC differentiation step is suitably performed in a predetermined period (e.g., approximately 2 days) following the Wnt signal-activating culture step, i.e., on 2 to 4 days (Day 2 to Day 4) counted from the start of induction of differentiation of the pluripotent stem cells mentioned above into a cell cluster having a predetermined micropattern.

For a culture method for allowing YSMCs to differentiate into EPCs, medium composition (the types of a basal medium, differentiation-inducing factors, other additives, etc. and the amounts of these used), culture conditions, and other items regarding culture, see Ohta, R. et al, J. Vis. Exp. (148), e59823, doi: 10.3791/59823 (2019), for example. For example, differentiation-inducing factors such as VEGF, FGF2 (bFGF), SCF, and a TGFb inhibitor (e.g., SB431542) can be added in appropriate amounts to a medium for the EPC differentiation step.

The cell population production method of the present invention can further comprise other steps, if necessary. Examples of such arbitrary steps include a culture step of further allowing EPCs obtained by the EPC differentiation step to differentiate into other cells.

In one embodiment of the present invention, the cell population production method of the present invention can further comprise a culture step of allowing EPCs to differentiate into CD34+CD32+ vitelline venous hemogenic endothelial cells (VVHECs) (VVHEC differentiation step). VVHECs are HECs derived from yolk sac mesoderm and are cells known to be responsible for hemogenesis in yolk sac in early fetal life. In the present invention, VVHECs are cells that can be obtained by the differentiation of YSMCs through EPCs. In addition to CD34 and CD32, FN1, ACTA2, and LYVE1 as well as venous markers such as NR2F2, APLNR and PROX1 are known as cell markers for VVHECs. VVHECs can be identified from whether to be positive to one or two or more (preferably all) thereof (positive cells are VVHECs, while negative cells are not VVHECs).

The VVHEC differentiation step is suitably performed in a predetermined period (e.g., approximately 2 days) following the EPC culture step, i.e., on 4 to 6 days (Day 4 to Day 6) counted from the start of induction of differentiation of the pluripotent stem cells mentioned above into a cell cluster having a predetermined micropattern.

For a culture method for allowing EPCs to differentiate into VVHECs, medium composition (the types of a basal medium, differentiation-inducing factors, other additives, etc. and the amounts of these used), culture conditions, and other items regarding culture, see Matsybara et al., Biochemical and Biophysical Research Communications, 515 (1), 2019, for example. For example, differentiation-inducing factors such as VEGF, SCF, Flt-3L, IL-3, IL-6, and TPO can be added in appropriate amounts to a medium for the VVHEC differentiation step.

The cell population production method of the present invention may further comprise a culture step of allowing VVHECs obtained by the VVHEC differentiation step to differentiate into other cells, for example, monocytes/macrophages (CD14-positive), or erythroid or myeloid progenitor cells (CD33-positive). A culture method for allowing VVHECs to differentiate into those cells, medium composition (the types of a basal medium, differentiation-inducing factors, other additives, etc. and the amounts of these used), culture conditions, and other items regarding culture can be the same as those for a method known in the art. In the case of allowing VVHECs to differentiate into monocytes/macrophages (CD14-positive), or erythroid or myeloid progenitor cells (CD33-positive), see Gene I. Uenishi et al., Nature Communications (2018) 9: 1828, for example.

### - Organoid or three-dimensional organ production method -

The organoid or three-dimensional organ production method of the present invention comprises the step of three-dimensionally culturing at least a portion of a cell cluster obtained by the cell cluster production method of the present invention, or a cell population obtained by the cell population production method of the present invention.

In the present specification, the term "three-dimensionally culturing" means that cells are cultured in a state embedded in a medium containing a component serving as a scaffold such as extracellular matrix. Various methods and embodiments are general or known in the art as organoid or three-dimensional organ production methods by three-dimensional culture (see, for example, Patent Literature 1 described above). In the present invention, an organoid or a three-dimensional organ can be produced by the same or similar method and embodiment as those for a conventional general production method or a conventional production method known in the art except that the cell cluster or the cell population according to the present invention is used as cells serving as a starting material for producing the organoid or the three-dimensional organ.

In recent years, methods for producing embryoid and gastruloid having a three-dimensionally constructed structure of early embryo, which are regarded as one type of organoid, have also been known in the art. Use of the cell cluster or the cell population according to the present invention containing cells, such as YSMCs or AECs, which are positioned in a boundary region between embryo and ectoderm has the possibility that embryoid or gastruloid that can more elaborately reproduce a structure can be produced.

### - Kit -

An alternative aspect of the present invention also provides a kit for producing a cell cluster enriched in desired cells from pluripotent stem cells by two-dimensionally culturing the pluripotent stem cells under Wnt signal regulation. The kit according to the present invention is, for example, a kit for the production of a cell cluster enriched in yolk sac mesoderm cells (YSMCs), comprising one or more Wnt signal activators, or a kit for the production of a cell cluster enriched in amniotic ectoderm cells (AECs), comprising one or more Wnt signal suppressors. Items regarding such a kit, for example, the Wnt signal activator, the Wnt signal suppressor, a culture method for use in each of the kits, and other constituent members that can be contained in each of the kits are the same or similar as the items described in relation to the cell cluster production method, the cell population production method, etc. in the present specification, and these described items can be referred to.

### - Definition -

### Pluripotent stem cell

In the present specification, the "pluripotent stem cells" refer to stem cells that can differentiate into tissues and cells having various different shapes and functions and have the ability to differentiate into cells of any lineage of the three germ layers (endoderm, mesoderm, and ectoderm). Examples of the pluripotent stem cells that may be used in the present invention include, but are not particularly limited to, induced pluripotent stem cells (also referred to as iPS cells), embryonic stem cells (also referred to as ES cells), embryonic stem cells derived from cloned embryos obtained by nuclear transplantation, spermatogonial stem cells, and embryonic germ cells.

The "induced pluripotent stem cells" (iPS cells) refer to cells that are obtained by reprograming mammalian somatic cells or undifferentiated stem cells by introducing particular factors (nuclear reprogramming factors). At present, there are various "induced pluripotent stem cells" and iPSCs established by Yamanaka, et al. by introducing the 4 factors Oct3/4, Sox2, Klf4, and c-Myc into mouse fibroblasts (Takahashi K, Yamanaka S., Cell, (2006) 126: 663-676); iPSCs derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors by a virus-free method (Okita K et al. Nat. Methods 2011 May; 8 (5): 409-12; and Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used. In addition, any of induced pluripotent stem cells known in the art described in all published non patent literatures (e.g., Shi Y., Ding S., et al., Cell Stem Cell, (2008) Vol. 3, Issue 5, 568-574; Kim JB., Scholer HR., et al., Nature, (2008) 454, 646-650; and Huangfu D., Melton, DA., et al., Nature Biotechnology, (2008) 26, No 7, 795-797) or patent literatures (e.g., Japanese Unexamined Patent Application Publication No. 2008-307007, Japanese Unexamined Patent Application Publication No. 2008-283972, US2008/2336610, US2009/047263, WO2007/069666, WO2008/118220, WO2008/124133, WO2008/151058, WO2009/006930, WO2009/006997, and WO2009/007852).

Various iPS cell lines established by NIH, Riken (the Institute of Physical and Chemical Research), Kyoto University, and the like may be used as the induced pluripotent stem cells (iPS cells). Examples of the human iPS cell lines include HiPS-RIKEN-1A line, HiPS-RIKEN-2A line, HiPS-RIKEN-12A line, and Nips-B2 line from Riken, and 201B7 line, 253G1 line, 253G4 line, 409B2 line, 454E2 line, 606A1 line, 610B1 line, 625A4 line, 648A1 line, 1201C1 line, 1205D1 line, 1210B2 line, 1231A3 line, 1383D2 line, and 1383D6 line from Kyoto University. Alternatively, for example, cell lines of clinical grade provided by Kyoto University, Cellular Dynamics International, and the like, and cell lines for research or clinical uses prepared using these cell lines may be used.

The "embryonic stem cells" (ES cells) that may be used include mouse ESCs such as various mouse ES cell lines established by inGenious Targeting Laboratory, Riken (the Institute of Physical and Chemical Research), and the like, and human ES cells such as various human ES cell lines established by NIH, Riken, Kyoto University, and Cellartis. For example, CHB-1 to CHB-12 lines, RUES1 line, RUES2 line, and HUES1 to HUES28 lines from NIH, H1 line and H9 line from WisCell Research, KhES-1 line, KhES-2 line, KhES-3 line, KhES-4 line, KhES-5 line, SSES1 line, SSES2 line, and SSES3 line from Riken can be used as the human ES cell lines. Alternatively, for example, cell lines of clinical grade and cell lines for research or clinical use prepared using these cell lines may be used.

### Cell marker

In the present specification, the "cell marker" refers to a gene that is specifically expressed (positive marker) or not specifically expressed (negative marker) in a predetermined cell type, specifically, a substance that is produced (positive marker) or not produced (negative marker) as mRNA through the transcription of the gene in the genome or as a protein through the translation of the mRNA. The cell marker is preferably capable of being labeled (stained) with a fluorescent material, and is a protein (cell surface marker) that is expressed on cell surface and facilitates the detection, concentration, isolation, or the like of cells expressing the cell marker.

The term "positive" to a marker gene means that the expression level of mRNA or a protein of the gene is detectable by an approach general or known to those skilled in the art, or is higher than a predetermined threshold (background level, etc.). The term "negative" to a marker gene means that the expression level of mRNA or a protein of the gene is not detectable by an approach general or known to those skilled in the art, or is lower than a predetermined threshold (background level, etc.).

Whether to be positive or negative to the cell marker can be determined from qualitative or quantitative results by an approach general or known to those skilled in the art. The cell marker as a protein can be detected or its expression level can be measured, by use of immunoassay using an antibody specific for the protein, for example, ELISA, immunostaining, flow cytometry, or the like. The cell marker as mRNA can be detected or its expression level can be measured, by use of assay using a nucleic acid specific for the mRNA, for example, RT-PCR (including quantitative PCR), a microarray, a biochip, or the like.

### Organoid

In the present specification, the "organoid" refers to a three-dimensional structure that is similar to any of various organs, tissues, and the like and is artificially created by combining plural types of cells. The organoid also encompasses, for example, organoids and cancer organoids and in the broad sense, also encompasses embryoid (e.g., Science 07 Jun 2019: Vol. 364, Issue 6444, pp. 948-951; DOI: 10.1126/science.aax0164) and gastruloid (e.g., Nature volume 582, pages 410-415 (2020)) having a three-dimensionally constructed structure of early embryo. The "organoid" encompasses not only organoids that are similar functionally and structurally to various organs or tissues and are at a relatively matured stage, but structures called "organ bud", "primordium", and the like which are at an early stage of such complication. For example, various types of organoids such as the liver, the pancreas, the kidney, the heart, the lung, the spleen, the esophagus, the stomach, the thyroid gland, the parathyroid gland, the thymus gland, the gonad, the brain, and the spinal cord are known in the art as the organoid (see, for example,
https://www.nejm.org/doi/pdf/10.1056/NEJMra1806175,
https://www.nature.com/articles/s41568-018-0007-6, and
http://www.amsbio.com/brochures/organoid-culture-handbook.pdf).

The three-dimensional structure of the organoid can be confirmed by visual or microscopic observation. The three-dimensional structure can be identified from whether to be positive to markers appropriate for cells contained in the organ, particularly, markers for parenchymal cells of the organ, more preferably, whether proteins of these markers are secreted into a culture supernatant, in addition to such confirmation of the three-dimensional structure.

### Three-dimensional organ

In the present specification, the "three-dimensional organ" can also be regarded as a mature organoid and refers to a structure containing a more mature cell population or structure than that of an organoid.

Whether the three-dimensional organ has been obtained from an organoid can be determined from one or more viewpoints, for example, the density of cells (e.g., whether to exceed a predetermined criterion) in a structure, the three-dimensional shape (e.g., whether to be more three-dimensional than a given level,) of a structure, a function or a trait (e.g., whether to have acquired a predetermined function or trait, such as a metabolic function), and a cell marker (e.g., whether to be positive to the expression of a gene or a protein of the cell marker, whether the density of the positive cells exceeds a predetermined criterion, or whether the amount of the marker protein secreted in a culture supernatant exceeds a predetermined criterion). The density of cells, the three-dimensional shape, the function or the trait, the cell marker, and the like described above can be appropriately set according to the organoid and the three-dimensional organ. The determination as described above can be based on, for example, whether to achieve a level equivalent to or similar to the organ *in vivo.*

The cells contained in the colony of pluripotent stem cells, the cell cluster, the cell population, the organoid, the three-dimensional organ, and the like according to the present invention may be derived from a human or may be derived from a nonhuman animal, for example, a mammal such as a mouse, a rat, a dog, a pig, or a monkey. When the organoid or the three-dimensional organ is used for the purpose of, for example, transplantation in a human or the development of a medicament for humans (e.g., the detection of a drug that causes drug addiction unlikely to be found by a conventional animal experiment, human cell test, or the like), the cells are preferably derived from a human.

In the present specification, an item described in a singular form and the item described in a plural form as to a cell, a compound, or other substances can be interpreted interchangeably with each other unless otherwise specified.

The term "comprise", "include", "contain", etc. refers to inclusion of the element(s) following the word without limitations thereto. Thus, this suggests inclusion of the element(s) following the word, but does not suggest exclusion of any other element. On the other hand, the term "consisting of", etc. means inclusion of every element following the term and a limitation thereto. Thus, the term "consist(s) of" or "consisting of" indicates that the enumerated element(s) is required or essential, and substantially no other elements exist. The term "consist(s) essentially of" or "consisting essentially of" means inclusion of any element following the term and a limitation of other elements to those influencing neither the activity nor the effect defined in the present disclosure as to the element following the term. Thus, the term "consisting essentially of", etc. indicates that the enumerated element(s) is required or essential, but other elements are optional and may or may not exist depending on whether to affect the activity or the effect of the enumerated element(s).

### Examples

In the following Examples, "Day" refers to the number of days from the start of induction of differentiation of a human iPS cell colony. "Day 0" is the start of culture under Wnt signal regulation.

### [Example 1] Preparation of yolk sac mesoderm cells and amniotic ectoderm cells under Wnt signal regulation

### [1] Preparation of human yolk sac mesoderm cells by Wnt signal-activating culture step (first embodiment of the present invention)

Human iPS cells (625A4; Center for iPS Cell Research and Application (CiRA), Kyoto University) were cultured at 37°C for 6 to 7 days under 5% CO₂ in AK02N (Ajinomoto Co., Inc.) (10 cm dish; 8 ml, 24-well plate; 0.5 ml) to form iPS cell colonies of 500 to 700 µm in diameter. The obtained colonies were cultured at 37°C for 2 days under 5% CO₂ in Essential 8 medium (Gibco) (10 cm dish; 8 ml, 24-well plate; 0.5 ml) supplemented with BMP4 (80 ng/ml), VEGF (80 ng/ml), and CHIR99021 (2 µM) (Days 0 to 2).

### [2] Preparation of human amniotic ectoderm cells by Wnt signal-inhibiting culture step (second embodiment of the present invention)

Human iPS cells (625A4; Center for iPS Cell Research and Application (CiRA), Kyoto University) were cultured at 37°C for 6 to 7 days under 5% CO₂ in AK02N (Ajinomoto Co., Inc.) (10 cm dish; 8 ml, 24-well plate; 0.5 ml) to form iPS cell colonies of 500 to 700 µm in diameter. The obtained colonies were cultured at 37°C for 2 days under 5% CO₂ in Essential 8 medium (Gibco) (10 cm dish; 8 ml, 24-well plate; 0.5 ml) supplemented with BMP4 (80 ng/ml), VEGF (80 ng/ml), and IWP-2 (2 µM) (Days 0 to 2).

iPS cell colonies were formed as a control in the same manner as in [1] and [2]. Then, the obtained colonies were cultured at 37°C for 2 days under 5% CO₂ in Essential 8 medium (Gibco) (10 cm dish; 8 ml, 24-well plate; 0.5 ml) supplemented with BMP4 (80 ng/ml) and VEGF (80 ng/ml) (Days 0 to 2).

### [3] Differentiation marker analysis of cell cluster by immunocytostaining

After induction of differentiation in the step of Days 0 to 2 in [1] or [2], the medium was removed, and the cells were washed once with PBS(-) (Gibco). Then, 4% paraformaldehyde was added at a volume of 500 µl/24 wells to the cells and left at room temperature for 15 minutes. Then, the cells were washed three times with PBS(-) and preserved overnight at 4°C. Then, PROTEIN BLOCK SERUM-FREE blocking solution [DAKO, X909] was added at a volume of 500 µl/24 wells to the cells and left at room temperature for 60 minutes for blocking. Then, a primary antibody (1/100 Anti-GATA6 (Abcam plc, ab22600), 1/50 Anti-Brachyury T (R&D Systems, Inc., AF2085), 1/400 Anti-Sox2 (CST, 3579P2), 1/100 Anti-CDX2 (R&D Systems, Inc., MAB3665), and 1/50 Anti-AP-2a (Santa Cruz Biotechnology Inc., sc-12726)) against the target antigen was added to a 1% blocking solution in PBS(-). The primary antibody solution was added at a volume of 500 µl/24 wells and left standing at room temperature for 60 minutes or overnight at 4°C. Subsequently, the cells were washed three times with PBS(-). Then, a secondary antibody (1/1000 Alexa Flour 555, 1/1000 Alexa Flour 647, and 1/1000 DAPI-HCB Hycult Biotech, HM2167) corresponding to the primary antibody was added to a 1% blocking solution in PBS(-). The secondary antibody solution was added at a volume of 500 µl/24 wells and incubated at room temperature for 60 minutes. Then, after washing three times with PBS(-), PBS(-) was added at a volume of 500 µl/24 wells and held. Fluorescent images were obtained under LSM880 confocal laser microscope (Zeiss). The processing of the obtained images and quantitative analysis were carried out using image analysis software "Fiji".

Figure 1 shows analysis results on the cell markers Brachyury, GATA6, and FOXF1 as to the cell cluster of Day 2 in [1]. It was confirmed that Brachyury-negative, GATA6-positive, and FOXF1-positive YSMCs were enriched, and in other words, a cell cluster having an increased YSMC region in an outer edge part was formed, when the Wnt signal activator CHIR99021 was added to a medium (+CHIR) in accordance with the first embodiment of the present invention, as compared with no addition of the Wnt signal activator (-) (Figures 1B and 1C). From images observed under an optical microscope, the cell cluster obtained by the first embodiment of the present invention was confirmed to also differ morphologically from the cell cluster obtained by a conventional method (Figure 1A). As shown in these drawings, predetermined cells in the cell cluster obtained by the method of the present invention are found to two-dimensionally form a clear layered structure.

Figure 2 shows analysis results on the cell markers GATA6, SOX2, CDX2, and TFAP2A as to the cell cluster of Day 2 in each of [1] and [2]. It was confirmed that CDX2-positive (middle box) and TFAP2A-positive (lower box) AECs were enriched, and in other words, a cell cluster having an AEC region in an outer edge part was formed, when the Wnt signal suppressor IWP-2 was added to a medium (+IWP-2) in accordance with the second embodiment of the present invention, as compared with no addition of the Wnt signal suppressor (-). Unlike the case of adding CHIR99021 to a medium (+CHIR), the addition of IWP-2 (+IWP-2) resulted in a small number of GATA6-positive cells in an outer edge part (upper box). It was also confirmed that under the condition of +CHIR, a CDX2-negative region (middle box) was GATA6-positive (upper box); and under the condition of +IWP-2, the CDX2-positive region (middle box) was GATA6-negative (upper box).

### [4] Preparation of human vitelline venous hemogenic endothelial cells

Following the preparation of human yolk sac mesoderm cells in [1], the medium was replaced with Essential 6 medium (Gibco) (10 cm dish; 8 ml) supplemented with VEGF (80 ng/ml), FGF2 (25 ng/ml), SCF (50 ng/ml), and SB431542 (2 µM), and hemangioblasts were induced by further culture at 37°C for 2 days under 5% CO₂. Then, the medium was replaced with Stempro-34 SFM (Gibco) (10 cm dish; 8 ml) supplemented with VEGF (80 ng/ml), SCF (50 ng/ml), Flt-3L (50 ng/ml), IL-3 (50 ng/ml), IL-6 (50 ng/ml), and TPO (5 ng/ml), and the cells were cultured at 37°C for 2 days under 5% CO₂. Then, the medium was replaced with a medium of the composition described above except for VEGF, and CD34-positive and CD32-positive vitelline venous hemogenic endothelial cells were induced by culture at 37°C for 2 days under 5% CO₂.

### [5] Differentiation marker analysis of cell population by immunocytostaining

After induction of differentiation of the step of Days 4 to 6 in [4], immunocytostaining was performed by the same procedures as in [3] except that the primary antibody used was changed to 1/50 Anti-CD34 (Abeam plc, ab81289). Figure 3(A) shows analysis results on cell markers Brachyury and CD34 as to the cell population of Day 6 in [4]. Rich CD34-positive vascular endothelial progenitor cells were confirmed to appear from a YSM region in the outer edge part of the cell cluster (+CHIR) obtained in accordance with the first embodiment of the present invention.

### [6] Differentiation marker analysis using flow cytometry

After induction of differentiation of vitelline venous hemogenic endothelial cells by the step of Days 6 to 8 in [4], the medium was removed, and the cells were washed twice with PBS(-) (Gibco). Then, TrypLE Express (Gibco) was added at a volume of 3 mL/dish and left at 37°C for 15 minutes. Then, the cells were recovered into a 15 mL tube while dissociated by pipetting with P1000 micropipette. After addition of 7 mL of Stempro-34 SFM (Gibco) medium, the cells were centrifuged at 1000 rpm at room temperature for 5 minutes. After removal of the supernatant, the cells were washed once using a PBS(-) buffer containing 1 mM EDTA and 4% FBS. Then, the cells were suspended in 50 µl of a primary antibody solution (1/50 PE-CD32 (BioLegend, Inc., Cat: 303205) and BV421-CD34 (BD Biosciences, Cat: 562577) in BD Horizon Brilliant(TM) Stain Buffer) and left standing on ice for 30 minutes. Subsequently, the cells were washed twice with a PBS(-) buffer containing 1 mM EDTA and 4% FBS, then suspended in a PBS(-) buffer containing 1/1000 DAPI, 1 mM EDTA, and 4% FBS, and transferred to a tube with a 40 µm cell strainer cap. The differentiation markers were analyzed by BD LSRFortessa flow cytometry using the obtained antibody-stained cell suspension. Data obtained from flow cytometry was analyzed using FlowJo.

Figure 3(B) shows results of flow cytometry as to the cell population of Day 8 in [4]. It was confirmed that more CD34-positive and CD32-positive vitelline venous hemogenic endothelial cells were produced in a YSM region in the outer edge part of the cell cluster (+CHIR) obtained in accordance with the first embodiment of the present invention, as compared with a control (-), and in other words, the CD34-positive vascular endothelial progenitor cells in the YSM region had the ability to further differentiate into CD32-positive vitelline venous hemogenic endothelial cells.

## Claims

1. A method for producing a cell cluster enriched in desired cells from pluripotent stem cells, the method comprising the step of two-dimensionally culturing the pluripotent stem cells under Wnt signal regulation.

2. The method according to claim 1, wherein the desired cells are yolk sac mesoderm cells, and the step of two-dimensionally culturing the pluripotent stem cells under Wnt signal regulation is a Wnt signal-activating culture step of two-dimensionally culturing the pluripotent stem cells under conditions that activate Wnt signals.

3. The method according to claim 2, wherein the Wnt signal-activating culture step is the step of two-dimensionally culturing the pluripotent stem cells using a medium supplemented with a Wnt signal activator.

4. The method according to claim 3, wherein the Wnt signal activator is a GSK3 inhibitor.

5. The method according to claim 2, wherein the Wnt signal-activating culture step is performed at the start of culture of the pluripotent stem cells.

6. The method according to claim 1, wherein the pluripotent stem cells are induced pluripotent stem cells.

7. A cell population production method further comprising a culture step of allowing the obtained yolk sac mesoderm cells to differentiate into CD34+ vascular endothelial progenitor cells after the Wnt signal-activating culture step according to claim 2.

8. The method according to claim 7, further comprising a culture step of allowing the CD34+ vascular endothelial progenitor cells to differentiate into CD34+CD32+ vitelline venous hemogenic endothelial cells.

9. A cell cluster obtained by the method according to claim 1.

10. A cell population obtained by the method according to claim 7.

11. An organoid or three-dimensional organ production method comprising the step of three-dimensionally culturing at least a portion of the cell cluster according to claim 9 or the cell population according to claim 10.

12. An organoid or a three-dimensional organ obtained by the production method according to claim 11.

13. The method according to claim 1, wherein the desired cells are amniotic ectoderm cells, and the step of two-dimensionally culturing the pluripotent stem cells under Wnt signal regulation is a Wnt signal-suppressing culture step of two-dimensionally culturing the pluripotent stem cells under conditions where Wnt signals have been suppressed.

14. The method according to claim 13, wherein the Wnt signal-suppressing culture step is performed using a medium supplemented with a Wnt signal inhibitor.

15. The method according to claim 14, wherein the Wnt signal inhibitor is IWP-2.
